(19) Europäisches Patentamt
European Patent Office
Office européen des brevets

(11) Publication number : **0 491 782 B1**

(12) **EUROPEAN PATENT SPECIFICATION**

(45) Date of publication of patent specification : **15.12.93 Bulletin 93/50**

(51) Int. Cl.$^5$ : **C12P 7/40,** C12P 7/00, // C07D301/16

(21) Application number : **90913693.9**

(22) Date of filing : **12.09.90**

(86) International application number : **PCT/DK90/00235**

(87) International publication number : **WO 91/04333 04.04.91 Gazette 91/08**

# (54) A PROCESS FOR PREPARING PEROXYCARBOXYLIC ACIDS USING AN ENZYME CATALYST.

(30) Priority : **12.09.89 DK 4503/89**
**20.07.90 DK 1737/90**

(43) Date of publication of application : **01.07.92 Bulletin 92/27**

(45) Publication of the grant of the patent : **15.12.93 Bulletin 93/50**

(84) Designated Contracting States : **AT BE CH DE DK ES FR GB IT LI LU NL SE**

(56) References cited :
**EP-A- 0 310 952**
**DE-A- 2 240 605**

(73) Proprietor : **NOVO NORDISK A/S**
**Novo Allé**
**DK-2880 Bagsvaerd (DK)**

(72) Inventor : **KIRK, Ole**
**Stefansgade 38, 3.tv.**
**DK-2200 Kobenhavn N (DK)**
Inventor : **BJÖRKLING, Frederik**
**Hövitsmansgatan 2**
**S-25237 Helsingborg (SE)**
Inventor : **GODTFREDSEN, Sven, Erik**
**Smedegade 15B**
**DK-3500 Vaerlose (DK)**

(74) Representative : **Noergaard, Torsten et al**
**c/o Novo Nordisk A/S Novo Allé**
**DK-2880 Bagsvaerd (DK)**

EP 0 491 782 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF INVENTION

The present invention relates to a novel process for the preparation of peroxycarboxylic acids. Also, the present invention relates to a process for the oxidation of organic compounds with the peroxycarboxylic acids thus prepared.

### BACKGROUND OF THE INVENTION

Peroxycarboxylic acids are commonly employed as oxidizing reagents in the field of organic synthesis for the production of organic chemicals. A wide variety of organic molecules may be oxidized by means of these reagents which, for instance, are useful for the preparation of epoxides from unsaturated hydrocarbons. The various uses of peroxycarboxylic acids are exhaustively described in the art, e.g. in Comprehensive Organic Chemistry edited by Barton and Ollis, Pergamon Press, 1979. Some peroxycarboxylic acids such as meta-chloro-peroxybenzoic acid have become commercially available as a result of their wide applicability.

Even though some peroxycarboxylic acids are useful and commercially available reagents their use is limited because of their relatively high cost and the risks (in particular the explosion hazard) involved in handling the reagents, especially when this is done on a large scale. Accordingly, there is a need for improved methods of preparing peroxycarboxylic acids as well as for methods which make it safer to use them in organic synthesis.

### DISCLOSURE OF THE INVENTION

It has surprisingly been found that peroxycarboxylic acids may be generated by treating carboxylic acids with hydrogen peroxide in the presence of an enzyme. This method of preparing peroxycarboxylic acids presents a safe and economically viable approach for the preparation of these highly useful reagents. It has also been found that enzymatic synthesis of peroxycarboxylic acids and oxidation of organic chemicals by means of peroxycarboxylic acids may be carried out simultaneously.

Accordingly, the present invention relates to a process for the preparation of a peroxycarboxylic acid of the general formula (I)

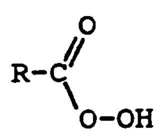

(I)

wherein R is an organic residue, in particular a linear or branched, saturated or unsaturated alkyl group, an aryl group or an alkyl aryl group each of which may optionally be substituted with one or more hydroxy, thio, alkyloxy, alcoxy, nitro, oxy, keto, oxo, halo or carboxy groups, the process comprising treating a carboxylic acid of the general formula (II)

R-COOH (II)

wherein R has the meaning indicated above, with hydrogen peroxide or a precursor thereof in the presence of an enzyme catalyst.

The process of the invention may be summarized as indicated in Reaction Scheme (A):

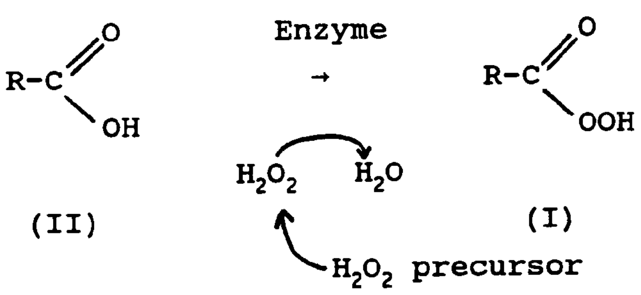


(II) (I)

(A)

In Reaction Scheme (A), R is as defined above. In particular, R may be alkyl with 1-30 carbon atoms, in

particular linear alkyl with 1-8 carbon atoms, or optionally substituted phenyl. If the carboxylic acid substrate (II) is an optically active carboxylic acid, one may employ a racemate or either of the enantiomer forms of the carboxylic acid substrate (II) in the process of the invention.

The enzyme catalyst employed in the process of the invention is preferably a hydrolytic enzyme such as a protease or a lipase. The suitability of a given enzyme for use in the present process may easily be tested by exposing a carboxylic acid substrate to hydrogen peroxide or a hydrogen peroxide precursor in the presence of the enzyme and by monitoring the generation of peroxycarboxylic acid from the reaction. The enzyme may be used as such, but it may also be chemically modified or immobilised in order to enhance its stability and its activity towards the substrate in question.

Lipases which may be employed in the present process may be microbial lipases produced, for instance, by strains of Aspergillus, Enterobacterium, Chromobacterium, Geotricium or Penicillium. Preferred lipases for use according to the invention are those produced by species of Mucor, Humicola, Pseudomonas or Candida.

Particularly preferred lipases are those produced by the following strains of microorganisms, all of which have been deposited in the Deutsche Sammlung von Mikroorganismen in accordance with the provisions of the Budapest Treaty on the International Recognition of the Deposit of Microorganisms for the purposes of Patent Procedure:

Candida antarctica, deposited on 29 September 1986, with the number DSM 3855, and on 8 December 1986, with the numbers DSM 3908 and DSM 3909.
Pseudomonas cephacia, deposited on 30 January 1987, with the number 3959.
Humicola lanuginosa, deposited on 13 August 1986 and 4 May, 1987 with the deposit numbers 3819 and 4109, respectively.
Humicola brevispora, deposited on 4 May 1987, with the deposit number DMS 4110,
Humicola brevis var. thermoidea, deposited on 4 May 1987, with the deposit number DSM 4111, and
Humicola insolens, deposited on 1 October 1981, with the deposit number DSM 1800.

Currently preferred lipases are those produced by Candida antarctica, DSM 3855, DSM 3908 and DSM 3909. These enzymes may be produced by the process disclosed in WO 88/02775. Briefly, the Candida strains in question may be cultivated under aerobic conditions in a nutrient medium containing assimilable carbon and nitrogen sources as well as essential minerals, trace elements etc., the medium being composed according to established practice. After cultivation, liquid enzyme concentrates may be prepared by removing insoluble materials, e.g. by filtration or centrifugation, after which the culture broth may be concentrated by evaporation or reverse osmosis. Solid enzyme preparations may be prepared from the concentrate by precipitation with salts or water-miscible solvents, e.g. ethanol, or by drying such as spray-drying in accordance with well-known methods.

Additional lipases may be obtained from the following strains which are publicly available without restriction from the Centraalbureau voor Schimmelculturen (CBS), American Type Culture Collection (ATCC), Agricultural Research Culture Collection (NRRL) and Institute of Fermentation, Osaka (IFO) with the following deposit numbers: Candida antarctica, CBS 5955, ATCC 34888, NRRL Y-8295, CBS 6678, ATCC 28323, CBS 6821 and NRRL Y-7954; Candida tsukubaensis, CBS 6389, ATCC 24555 and NRRL Y-7795;Candida auriculariae, CBS 6379, ATTC 24121 and IFO 1580; Candida humicola, CBS 571, ATCC 14438, IFO 0760, CBS 2041, ATCC 9949, NRRL Y-1266, IFO 0753 and IFO 1527; and Candida foliorum, CBS 5234 and ATCC 18820.

It is known to produce lipase by recombinant DNA techniques, cf. for instance EP 238 023. Recombinant lipases may also be employed for the present purpose.

When employed in the process of the invention, the enzyme may be in a soluble state. It is, however, preferred to immobilize the enzyme in order to facilitate the recovery of the peroxycarboxylic acids produced by the present process. Immobilization procedures are well known (cf. for instance K. Mosbach, ed., "Immobilized Enzymes", Methods in Enzymology 44, Academic Press, New York, 1976) and include cross-linking of cell homogenates, covalent coupling to insoluble organic or inorganic supports, entrapment in gels and adsorption to ion exchange resins or other adsorbent materials. Coating on a particulate support may also be employed (cf. for instance A.R. Macrae and R.C. Hammond, Biotechnology and Genetic Engineering Reviews 3, 1985, p. 193. Suitable support materials for the immobilized enzyme are, for instance, plastics (e.g. polypropylene, polystyrene, polyvinylchloride, polyurethane, latex, nylon, teflon, dacron, polyvinylacetate, poly-vinylalcohol or any suitable copolymer thereof), polysaccharides (e.g. agarose or dextran), ion exchange resins (both cation and anion exchange resins), silicon polymers (e.g. siloxane) or silicates (e.g. glass).

It is preferred to immobilize the enzyme on an ion exchange resin by adsorbing the enzyme to the resin or by cross-linking it to the resin by means of glutaraldehyde or another cross-linking agent in a manner known per se. A particularly preferred resin is a weakly basic anion exchange resin which may be a polystyrene-, acrylic- or phenol-formaldehyde-type resin. Examples of commercially available polyacrylic-type resins are Lewatit E 1999/85 (registered trademark of Bayer, Federal Republic of Germany) and Duolite ES-568 (registered

trademark of Rohm & Haas, FRG). Immobilization of enzymes to this type of resin may be carried out according to EP 140 542. Immobilization to phenyl-formaldehyde-type resins may be done according to DK 85/878. An example of a commercially available acrylic-type resin is Lewatit E2001/85 (registered trademark of Bayer, FRG).

Another convenient material for immobilizing enzymes is an inorganic support, such as a silicate. The enzyme may be attached to the support by adsorption or by covalent coupling, eg. as described in K. Mosbach, ed., op.cit.

The process according to the method of the invention may be carried out in the carboxylic acid substrate itself (which in this case also acts as a solvent) or in solvents such as water, aqueous buffer solutions or organic solvents. Some preferred organic solvents are hydrocarbons such as hexane, cyclohexane, heptane, benzene and toluene, methylene chloride and hexachloroethene, or acetonitrile, dimethylformamide, dioxane and tetrahydrofurane. It is preferred to employ solvents in which the substrate and products of the reaction are highly soluble and in which the enzyme maintains a good stability and activity.

If an organic substance e.g. an alkene is to be oxidized in situ by the Peroxycarboxylic acid being formed, this substance may advantageously be used as the solvent.

The temperature at which the reaction of the carboxylic acid (II) takes place is not critical, but is conveniently in the range of about 20-100 °C, such as about 30-80 °C.

The hydrogen peroxide employed according to the process of the invention may be added as such to the reaction mixture either at the beginning of the reaction or at a desired rate in the course of the reaction. Alternatively, it is possible to employ a precursor of hydrogen peroxide such precursors being compounds which give rise to the formation of hydrogen peroxide in situ, such as percarbonates or perborates.

The water generated in the course of the process according to the invention may, if desired, be removed by methods known in the art such as by distillation, exposure to dessicants etc.

As indicated above, it has surprisingly been found that the process of the present invention may be carried out concomitantly with oxidation of an organic compound susceptible to oxidation with peroxycarboxylic acids as indicated in Reaction Scheme (B) below:

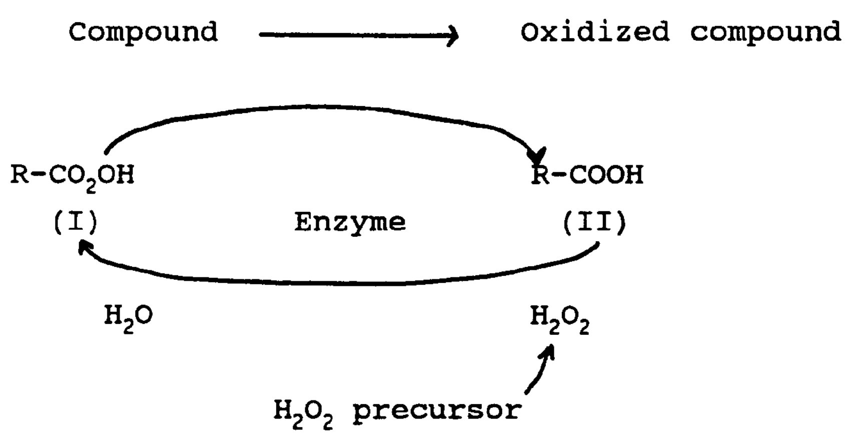


(B)

In Reaction Scheme (B) the "compound" may be any compound susceptible to peroxycarboxylic acid oxidation, e.g. an unsaturated hydrocarbon or a sulphur-containing organic molecule, while "R", "Enzyme" and "$H_2O_2$-precursor" have the meanings indicated above.

The process of the invention shown in Reaction Scheme (B) above may be carried out using substantially equimolar amounts of the "compound" and the carboxylic acid substrate (II). If desired, however, the process may be carried out using less than equimolar amounts of the carboxylic acid (II) since this compound will be regenerated in the course of the reaction as indicated in Reaction Scheme (B).

The process shown in Reaction Scheme (B) makes it unnecessary to transport and handle large quantities of peroxycarboxylic acids so that the hazards involved in using these oxidants are greatly reduced.

Furthermore, as indicated above, the carboxylic acid (II) may be prochiral or chiral and, in such instances, employed as a racemate or as either of its optically active stereoisomers. In cases, where the "compound" is prochiral or chiral, the carboxylic acid substrate (II) and the enzyme may be so selected that the "oxidized compound" is generated in a desired optically active form or as a racemate. The process of the invention may thus be employed for the synthesis of optically active compounds.

The process shown in Reaction Scheme (B) has for instance been found to be advantageous in the preparation of epoxides from alkenes.

The process of the invention is further illustrated in the following non-limiting examples.

General procedures :

Peroxycarboxylic acid references were prepared according to W. E. Parker, C. Ricciuti, C. L. Ogg and D. Swern, J. Am. Chem. Soc. 77, 4037 (1955). Peroxycarboxylic acid production was monitored by means of HPLC (Merck LiChrosorb RP-18 column, MeOH/$H_2O$/HCOOH ; 70/30/0.1, and UV-detector). Reactions were monitored by means of capillary gas chromatography.

Example 1.

To a stirred solution of octanoic acid (720 mg, 5 mmol) in hexane (50 ml) was added immobilized lipase derived from Candida antarctica (1 g, prepared according to examples 1 and 19 of WO 88/02775) and hydrogen peroxide (170 mg, 5 mmol, added as 510 µl 30 % solution). After the reaction mixture had been refluxed for 30 min, HPLC analysis showed a peroxyoctanoic acid concentration of 43 mM (43 % conversion).

Example 2.

Octanoic acid was oxidized as described in example 1 with the exception that acetonitrile was used as the solvent. After 30 min, 15 mM peroxyoctanoic acid was detected.

Example 3.

Example 1 was repeated in the presence of either 10 mM styrene or 10 mM 1,2-epoxyethylbenzene. In both cases, a peroxycarboxylic acid production which was comparable to that of example 1 was observed. Thus the enzyme did not appear to be inhibited by either of these compounds.

Example 4:

Preparation of styrene oxide:
To a stirred suspension (at room temperature) of immobilized C. antartica lipase (370 mg, according to example 1) in styrene (4.15 ml, 36.1 mmol) containing myristic acid (823 mg, 3.6 mmol) hydrogen peroxide (3 ml, 52.5 mmol) was added in six equal portions after 0, 0.5, 1.0, 1.5, 2.0 and 3.0 hours. After 1.5 hours 16.5% styrene oxide was formed as determined by gas chromatography.

Example 5.

Peroxycarboxylic acids were prepared from various carboxylic acids (see Table 1) as follows:
To a solution of carboxylic acid (630mM, 3.15 mmol) in toluene (5 ml) was added immobilized C. antarctica lipase (100 mg, as described in example 1) and hydrogen peroxide (255 µl, 60% (w/v), 4.45 mmol), with stirring. The reaction was allowed to proceed at room temperature for 120 minutes after which the yield of peroxycarboxylic acid was determined by RP-18 HPLC. The results are shown in Table 1 below.

Table 1

| Carboxylic acid | mM peroxycarboxylic acid |
|---|---|
| Hexanoic acid | 228 |
| Octanoic acid | 236* |
| Decanoic acid | 249 |
| Dodecanoic acid | 270 |
| Tetradecanoic acid | 277 |
| Hexadecanoic acid | 271 |
| Octadecanoic acid | 170 |

* In this case, xylene was used as the solvent due to interference by toluene in the assay.

Example 6

Epoxides were prepared from various alkenes (cf. Table 2) as follows:

To a stirred suspension of immobilized C. antarctica lipase (100 mg, as described in example 1) in a solution of hexane (10 ml), alkene (10mM) and octanoic acid (10mM) was added hydrogen peroxide (25µl, 0.05 mmol, 60%) at room temperature. The reaction was allowed to proceed for 4 hours after which the yield of epoxide was determined by capillary gas chromatography.

To compare, 10mM preformed peroxyoctanoic acid (prepared as described in D. Sweern, Organic Peroxides 2, Ch. 5, Wiley Interscience, New York, 1971; GB 1 452 730; W.E. Parker et al., J. Amer. Chem. Soc. 79, 1957 p. 1929) was added to 10 ml of a 10 mM solution of alkene in hexane. The reaction time in this experiment was also 4 hours. The yield of epoxide was determined as indicated above.

The results are shown in Table 2 below.

Table 2

| | % Yield | |
|---|---|---|
| Alkene | Catalytic oxidation | Chemical oxidation |
| Cyclohexene | 2 | 10 |
| 3-Ethyl-2-pentene | 48 | 50 |
| Tetramethylethylene | 99 | 74 |

Example 7

Epoxides were prepared from various alkenes (cf. Fig. 1) in a solvent-free medium as follows:

To a stirred suspension of immobilized C. antarctica lipase (175 mg, as described in example 1) in alkene (1.75 ml, about 15 mmol) containing octanoic acid (270 µl, 1.7 µmol) was added hydrogen peroxide (235 µl, 60% w/v) at the beginning of the reaction and after 1, 1.5, 2.5 and 4 hours (20 mmol in all). Yields were determined by capillary gas chromatography.

The results are shown in Fig. 1, in which ◊ is cyclohexene, x is 3-ethyl-2-pentene, ∇ is tetramethylethylene, and □ is 1-octene.

Example 8:

Preparation of hexadecene oxide, method A :

A mechanicaly stirred suspension of immobilized C. antartica lipase (1 g, according to example 1) in 1-hexadecene (15 ml, ca. 52.4 mmol) containing myristic acid (1.2 g, 5.25 mmol) was heated to 50°C. To this mixture hydrogen peroxide (4.6 ml 60% (w/v)) was added in four equal portions after 0, 1.5, 3 and 4.5 hours (80.5 mmol in all). After 20 hours reaction time 51% hexadecene oxide was formed as determined by gas chromatography.

$^1$H NMR: δ= 2.88-2.93 (m, 1H), 2.72-2.76 (dd, 1H), 2.45-2.49 (dd, 1H), 1.5-1.55 (m, 2H), 1.42-1.48 (m, 2H), 1.25-1.32 (m, 22H), 0.89 (t, 3H)

Example 9:

Preparation of hexadecene oxide, method B:

A mechanicaly stirred suspension of immobilized C. antartica lipase (1 g, according to example 1) in 1-hexadecene (15 ml, ca. 52.4 mmol) containing myristic acid (3.0 g, 13.1 mmol) was heated to 50°C. To this mixture hydrogen peroxide (4.6 ml 60% (w/v)) was added in four equal portions after 0, 1.5, 3 and 4.5 hours (80.5 mmol in all). After 24 hours reaction time 79.4 % hexadecene oxide was formed as determined by gas chromatography.

$^1$H NMR: δ= 2.88-2.93 (m, 1H), 2.72-2.76 (dd, 1H), 2.45-2.49 (dd, 1H), 1.5-1.55 (m, 2H), 1.42-1.48 (m, 2H), 1.25-1.32 (m, 22H), 0.89 (t, 3H)

Example 10:

Preparation of octene oxide, method A:

A mechanichaly stirred suspension of immobilized C. antartica lipase (100 mg, according to example 1) in toluene (13.5 ml), 1-octene (1.5 ml, 9.54 mmol) and myristic acid (0.22 g, 0.95 mmol) was heated to 50°C. To this mixture hydrogen peroxide (0.8 ml, 60% (w/v)) was added in four equal portions after 0, 1.5, 3 and 4.5 hours (14 mmol in all). After 24 hours 35% octene oxide was formed as determined by gas chromatography.

$^1$H NMR: δ= 2.87-2.96 (m, 1H), 2.73-2.78 (dd, 1H), 2.45-2.49 (dd, 1H), 1.5-1.57 (m, 2H), 1.43-1.49 (m, 2H), 1.27-1.39 (m, 6H), 0.9 (t, 3H)

Example 11:

Preparation of octene oxide, method B:

To a mechanicaly stirred suspension (50°C) of immobilized C. antartica lipase (1 g, according to exemple 1) in 1-octene (15 ml, ca.95.4 mmol) containing myristic acid (4.356 g, 19.1 mmol) was hydrogen peroxide (8 ml 60% (w/v)) added in four equal portions after 0, 1.5, 3 and 4.5 hours (140 mmol in all). After 6 hours 51% octene oxide was formed as determined by gas chromatography.

$^1$H NMR: δ= 2.87-2.96 (m, 1H), 2.73-2.78 (dd, 1H), 2.45-2.49 (dd, 1H), 1.5-1.57 (m, 2H), 1.43-1.49 (m, 2H), 1.27-1.39 (m, 6H), 0.9 (t, 3H)

Example 12:

Preparation of methylene cyclohexane oxide :

To a stirred suspension (at room temperature) of immobilized C. antartica lipase (24 mg, according to example 1) in toluene (3 ml), methylene cyclohexane (0.24 ml, 2 mmol) and myristic acid (46mg), was hydrogen peroxide (0.175 ml 60% (w/v)) added in five equal portions after 0, 1, 2, 3 and 4 hours. After 24 hours 97% methylene cyclohexane oxide was formed as determined by gas chromatography.

Example 13:

Preparation of penicillin V sulfoxide :

To a mechanicaly stirred suspension of immobilized C. antartica lipase (150 mg, according to example 1) in toluene (15 ml) and myristic acid (1 g, 4.6 mmol), was hydrogen peroxide (1.2 ml 60% (w/v), 21 mmol in all) added in four equal portions after 0, 2, 3 and 4 hours. Penicillin V-potassium salt (2.4 g, 6.2 mmol in all) was added in three equal portions after 2, 3 and 4 hours. The yields were determined by HPLC (Column: Supelco RP-18 ,5 micro; Mobile phase: Acetonitrile 20% and pH 6.5 Phosphate buffer 80%; Flow: 1ml/min. UV-detector). Full turnover was reached after 5 hours total reaction time.

Example 14:

Preparation of didodecane disulfide:
To a suspension of immobilized C.antartica lipase (100 mg, according to example 1) in toluene (5 ml), dodecanthiol (1.18 ml, 4.9 mmol) and myristic acid (1.12 g) was added hydrogen peroxide (0.28 ml 60% (w/v)). Product formed (Tlc, ethylacetate).

Example 15:

Preparation of δ-valerolactone:
To a stirred suspension (at room temperature) of immobilized C. antartica lipase (100 mg, according to example 1) in toluene (3 ml), cyclopentanon (0.265 ml, 3 mmol) and myristic acid (228 mg), was hydrogen peroxide (0.5 ml 60% w/v, 8.75 mmol) added. After 20 hours 16% δ-valerolactone was formed as determined by gas chromatography.

## Claims

1. A process for the preparation of a peroxycarboxylic acid of the general formula (I)

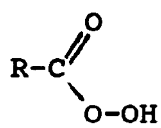

(I)

wherein R is an organic residue, in particular a linear or branched alkyl group, an aryl group or an alkyl aryl group each of which is optionally substituted with one or more hydroxy, thio, alkyloxy, alcoxy, nitro, oxy, keto, oxo, halo or carboxy groups, the process comprising treating a carboxylic acid of the general formula (II)

R-COOH (II)

wherein R has the meaning indicated above, with hydrogen peroxide or a precursor thereof in the presence of an enzyme catalyst.

2. A process according to claim 1, wherein the enzyme catalyst is a hydrolase.

3. A process according to claim 2, wherein the hydrolase is a protease or a lipase.

4. A process according to claim 3, wherein the enzyme is a microbial enzyme.

5. A process according to claim 4, wherein the enzyme is a bacterial enzyme.

6. A process according to claim 5, wherein the lipase is one obtainable from a species of Pseudomonas.

7. A process according to claim 6, wherein the lipase is one obtainable from Pseudomonas cephacia, DSM 3959.

8. A process according to claim 4, wherein the enzyme is a fungal enzyme.

9. A process according to claim 8, wherein the lipase is one obtainable from a species of Mucor, Aspergillus, Humicola, or Candida.

10. A process according to claim 9, wherein the lipase is one obtainable from Candida antarctica, DSM 3855, DSM 3908 or DSM 3909, Humicola lanuginosa, DSM 3819 or DSM 4109, Humicola brevispora, DSM 4110, Humicola brevis var. thermoidea, DSM 4111, or Humicola insolens, DSM 1800.

11. A process according to any of the preceding claims, wherein the enzyme is an immobilized enzyme.

12. A process according to any of the preceding claims, wherein the reaction is carried out in an organic solvent.

**13.** A process according to any of the preceding claims, wherein R is alkyl with 1-30 carbon atoms, in particular linear alkyl with 1-8 carbon atoms, or optionally substituted phenyl.

**14.** A process according to any of the preceding claims, wherein R is chiral or prochiral.

**15.** A process comprising the following steps:
a) preparation of peroxycarboxylic acid according to claim 1
b) the use of the peroxycarboxylic acid obtained in step a) for oxidation of organic compounds

**16.** A process according to claim 15, wherein the peroxycarbolxylic acid is generated in situ.

**17.** A process according to claim 15 or 16, wherein the organic compound is an alkene.

**18.** A process according to any of the claims 15 to 17, wherein the process is carried out using one or more of the reactants as solvent.

## Patentansprüche

**1.** Verfahren zur Herstellung einer Peroxycarbonsäure der allgemeinen Formel (I)

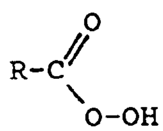

(I)

wobei R ein organischer Rest ist, insbesondere eine lineare oder verzweigte Alkylgruppe, eine Arylgruppe oder eine Alkylarylgruppe, von denen jede fakultativ mit einer oder mehreren Hydroxy-, Thio-, Alkyloxy-, Alkoxy-, Nitro-, Oxy-, Keto-, Oxo-, Halo- oder Carboxygruppen substituiert ist, wobei das Verfahren Behandlung einer Carbonsäure der allgemeinen Formel (II)

R-COOH (II)

wobei R die oben angegebene Bedeutung hat, mit Wasserstoffperoxid oder einer Vorstufe desselben in der Gegenwart eines Enzymkatalysators umfaßt.

**2.** Verfahren nach Anspruch 1, wobei der Enzymkatalysator eine Hydrolase ist.

**3.** Verfahren nach Anspruch 2, wobei die Hydrolase eine Protease oder eine Lipase ist.

**4.** Verfahren nach Anspruch 3, wobei das Enzym ein mikrobielles Enzym ist.

**5.** Verfahren nach Anspruch 4, wobei das Enzym ein bakterielles Enzym ist.

**6.** Verfahren nach Anspruch 5, wobei die Lipase eine ist, die aus einer Spezies von Pseudomonas gewonnen werden kann.

**7.** Verfahren nach Anspruch 6, wobei die Lipase eine ist, die aus Pseudomonas cephacia, DSM 3959, gewonnen werden kann.

**8.** Verfahren nach Anspruch 4, wobei das Enzym ein Pilzenzym ist.

**9.** Verfahren nach Anspruch 8, wobei die Lipase eine ist, die aus einer Spezies von Mucor, Aspergillus, Humicola oder Candida gewonnen werden kann.

**10.** Verfahren nach Anspruch 9, wobei die Lipase eine ist, die aus Candida antarctica, DSM 3855, DSM 3908 oder DSM 3909, Humicola lanuginosa, DSM 3819 oder DSM 4109, Humicola brevispora, DSM 4110, Humicola brevis var. thermoidea, DSM 4111 oder Humicola insolens, DSM 1800, gewonnen werden kann.

11. Verfahren nach einem der vorangehenden Ansprüche, wobei das Enzym ein immobilisiertes Enzym ist.

12. Verfahren nach einem der vorangehenden Ansprüche, wobei die Reaktion in einem organischen Lösungsmittel durchgeführt wird.

13. Verfahren nach einem der vorangehenden Ansprüche, wobei R Alkyl mit 1-30 Kohlenstoffatomen, insbesondere lineares Alkyl mit 1-8 Kohlenstoffatomen, oder fakultativ substituiertes Phenyl ist.

14. Verfahren nach einem der vorangehenden Ansprüche, wobei R chiral oder prochiral ist.

15. Verfahren, welches die folgenden Schritte umfaßt:
    a) Herstellung einer Peroxycarbonsäure gemäß Anspruch 1,
    b) die Verwendung der in Stufe a) erhaltenen Peroxycarbonsäure zur Oxidation von organischen Verbindungen.

16. Verfahren nach Anspruch 15, wobei die Peroxycarbonsäure in situ erzeugt wird.

17. Verfahren nach Anspruch 15 oder 16, wobei die organische Verbindung Alken ist.

18. Verfahren nach einem der Ansprüche 15 bis 17, wobei das Verfahren unter Verwendung einer oder mehrerer der Reaktanten als Lösungsmittel durchgeführt wird.

## Revendications

1. Procédé pour la préparation d'un acide peroxycarboxylique de la formule générale (I)

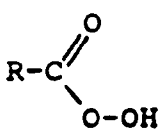

(I)

dans laquelle R est un résidu organique, en particulier un groupe alkyle linéaire ou ramifié, un groupe aryle ou un groupe alkylaryle dont chacun est facultativement substitué par un ou plusieurs groupes hydroxy, thio, alkyloxy, alcoxy, nitro, oxy, céto, oxo, halo ou carboxy, le procédé comprenant le traitement d'un acide carboxylique de formule générale (II)

R-COOH (II)

dans laquelle R a la signification indiquée ci-dessus, avec du peroxyde d'hydrogène ou son précurseur en présence d'un catalyseur enzymatique.

2. Procédé selon la revendication 1, dans lequel le catalyseur enzymatique est une hydrolase.

3. Procédé selon la revendication 2, dans lequel l'hydrolase est une protéase ou une lipase.

4. Procédé selon la revendication 3, dans lequel l'enzyme est une enzyme microbienne.

5. Procédé selon la revendication 4, dans lequel l'enzyme est une enzyme bactérienne.

6. Procédé selon la revendication 5, dans lequel la lipase est une lipase pouvant être obtenue à partir d'une espèce de Pseudomonas.

7. Procédé selon la revendication 6, dans lequel la lipase est une lipase pouvant être obtenue à partir de Pseudomonas cephacia, DSM 3959.

8. Procédé selon la revendication 4, dans lequel l'enzyme est une enzyme fongique.

9. Procédé selon la revendication 8, dans lequel la lipase est une lipase pouvant être obtenue à partir d'une espèce de Mucor, Aspergillus, Humicola, ou Candida.

10. Procédé selon la revendication 9, dans lequel la lipase est une lipase pouvant être obtenue à partir Candida antartica, DSM 3855, DSM 3908 ou DSM 3909, Humicola lanuginosa, DSM 3819 ou DSM 4109, Humicola brevispora, DSM 4110, Humicola brevis var. thermoidea, DSM 4111, ou Humicola insolens, DSM 1800.

11. Procédé selon l'une quelconque des revendications précédentes, dans lequel l'enzyme est une enzyme immobilisée.

12. Procédé selon l'une quelconque des revendications précédentes, dans lequel la réaction est effectuée dans un solvant organique.

13. Procédé selon l'une quelconque des revendications précédentes, dans lequel R est alkyle avec 1-30 atomes de carbone, en particulier un alkyle linéaire avec 1-8 atomes de carbone ou facultativement substitué phényle.

14. Procédé selon l'une quelconque des revendications précédentes, dans lequel R est chiral ou prochiral.

15. Procédé comprenant les étapes suivantes :
   a) préparation d'acide peroxycarboxylique selon la revendication 1
   b) utilisation de l'acide peroxycarboxylique obtenu à l'étape a) pour l'oxydation du composé organique.

16. Procédé selon la revendication 15, dans lequel l'acide peroxycarboxylique est produit in situ.

17. Procédé selon la revendication 15 ou 16, dans lequel le composé organique est un alcène.

18. Procédé selon l'une quelconque des revendications 15 à 17, dans lequel le procédé est effectué en utilisant un ou plusieurs des réactifs comme solvant.

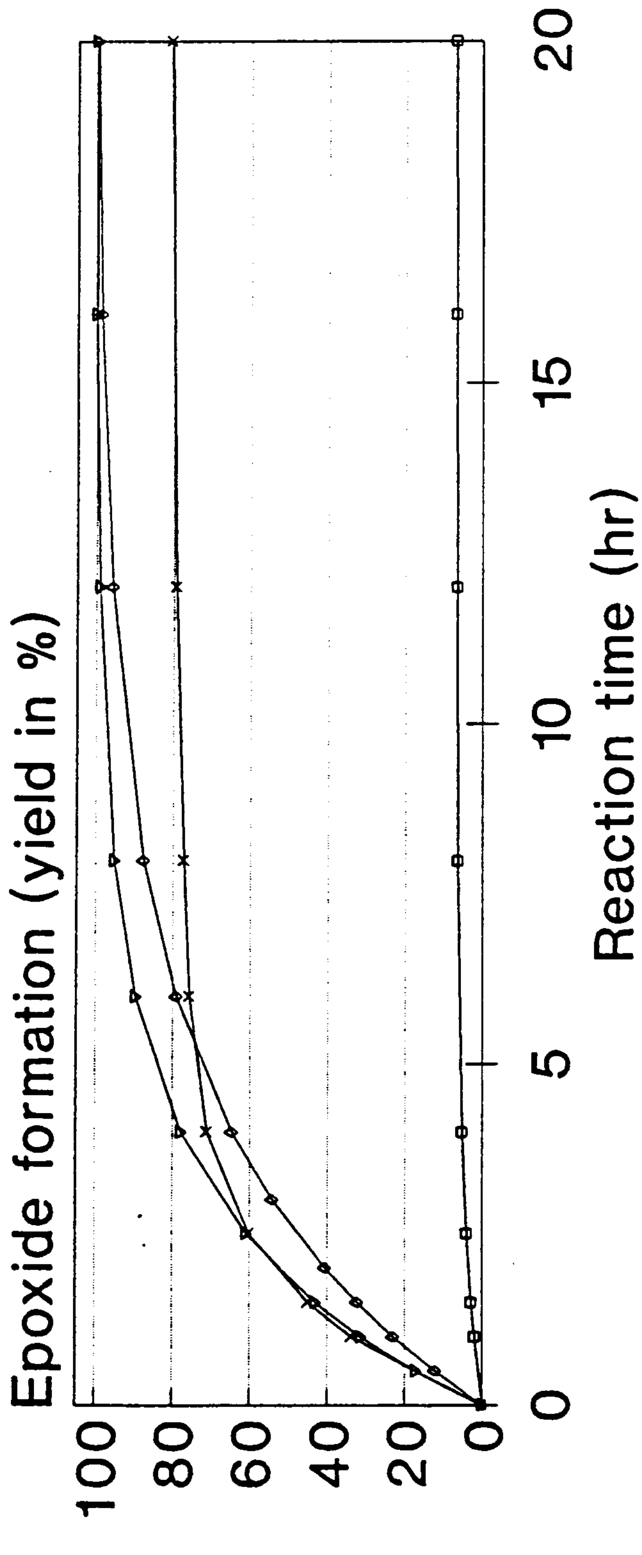
Epoxide formation (yield in %)
100
80
60
40
20
0
0
5
10
15
20
Reaction time (hr)

Figure 1